# FASCICULE DE BREVET EUROPEEN

(11) **EP 0 705 599 B1**
(45) Date de publication et mention de la délivrance du brevet: **25.09.1996**
(21) Numéro de dépôt: 95401937.8
(22) Date de dépôt: 23.08.1995
(51) Int. Cl.: A61K 7/22, A61K 7/50

(54) **Utilisation de N-(hydroxyalkyl) carbamates d'alkyle dans une composition cosmétique en tant que tensioactifs**
Verwendung von N-(hydroxyalkyl)-substituierten Alkylcarbamaten als Tensid in kosmetischen Zusammensetzungen
Use of N-(hydroxyalkyl)-substituted alkyl carbamates as tensioactive agents in cosmetic composition

(30) Priorité: 29.09.1994 FR 9411665
(43) Date de publication de la demande: 10.04.1996
(73) Titulaire: L'OREAL, F-75008 Paris (FR)
(72) Inventeur: Ascione, Jean-Marc, F-75018 Paris (FR); Bollens, Eric, F-94130 Nogent sur Seine (FR); Mahieu, Claude, F-75017 Paris (FR); Philippe, Michel, F-91320 Wissous (FR); Rollat-Corvol, Isabelle, F-75017 Paris (FR)
(74) Mandataire: Dodin, Catherine

(56) Documents cités:
- EP-A- 0 408 448
- EP-A- 0 420 722
- EP-A- 0 450 527
- EP-A- 0 577 506
- EP-A- 0 666 251
- EP-A- 0 677 288
- BE-A- 543 132
- FR-A- 2 379 283
- US-A- 2 040 997
- US-A- 2 808 402

## Description

La présente invention concerne l'utilisation d'un N-(hydroxyalkyl)carbamate d'alkyle dans une composition cosmétique ou pharmaceutique.

On connaît les N-(hydroxyalkyl)carbamates d'alkyle de formule : 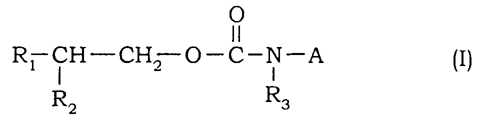 dans laquelle:
- R₁ représente un radical alkyle, saturé ou insaturé, linéaire ou ramifié, ayant 4 à 6 atomes de carbone,
- R₂ représente un radical alkyle, saturé ou insaturé, linéaire ou ramifié, ayant 2 à 4 atomes de carbone,
- R₃ représente un atome d'hydrogène ou un radical alkyle, saturé ou insaturé, linéaire ou ramifié, ayant 1 à 6 atomes de carbone, et
- A représente un groupement hydrophile non ionique.

Il est connu que ces composés présentent d'excellentes propriétés cosmétiques. En particulier, utilisés dans des compositions capillaires, ils confèrent aux cheveux des effets coiffant, lissant et gainant et facilitent leur démêlage.
De plus, il est connu que certains des composés de formule (I) permettent de diminuer la perte en eau et/ou d'augmenter la fixation d'eau dans le stratum corneum, et trouvent donc une application dans le domaine des compositions hydratantes.

La demanderesse a constaté que, de manière surprenante, les composés selon la formule (I) présentaient également des propriétés tensioactives intéressantes, susceptibles d'être utilisées dans le domaine cosmétique
Il a plus particulièrement été constaté que les composés de formule (I) présentaient des propriétés tensioactives « douces », c'est-à-dire nettement moins irritantes pour la peau ou le cuir chevelu que les tensioactifs habituellement employés.

La présente invention a donc pour objet l'utilisation d'un composé de formule (I) en tant que tensioactif dans une composition cosmétique ou pharmaceutique.

Par composition cosmétique, on entend dans la présente description, toute composition susceptible d'être appliquée entre autre sur la peau ou les cheveux, et comprenant habituellement un tensioactif, les compositions bucco-dentaires étant également comprises dans cette définition.

L'invention présente également comme avantage d'utiliser des composés présentant un pouvoir tensioactif important, supérieur à celui de composés de structure proche connus pour être de bons tensioactifs. Il en résulte la possibilité d'obtenir des effets similaires tout en employant moins de composé tensioactif.

Parmi les composés de formule (I) tel que définis ci-dessus, on préfère ceux pour lesquels A représente un radical :

―(CH₂)ₙ―(CHOH)ₘ―Z

dans lequel n est un nombre entier égal à 0 ou 1, m est un nombre entier compris entre 0 et 4, et Z est un radical alkyle hydroxylé ayant de 1 à 4 atomes de carbone.
Par radical alkyle hydroxylé, on entend un radical alkyle, sature ou insaturé, ramifié ou linéaire, contenant 1 à 3 groupements hydroxyles.
En particulier, Z peut être choisi dans le groupe comprenant :

―CH₂OH et ―C(CH₂OH)₃

Parmi les composés de formule 1, on peut citer tout particulièrement :
- la N-2-éthylhexyloxycarbonyl-N-méthyl-D-glucamine
- la N-2-butyloctyloxycarbonyl-N-méthyl-D-glucamine,
- la N-2-éthylhexyloxycarbonyl-D-glucamine,
- la N-2-butyloctyloxycarbonyl-D-glucamine,
- le 2-N-(2-éthylhexyloxycarbonyl)-amino-2-hydroxyméthyl-1,3-propanediol,
- le 2-N-(2-butyloctyloxycarbonyl)-amino-2-hydroxyméthyl-1,3-propanediol,
- le 2-N-(2-éthylhexyloxycarbonyl)-amino-1-éthanol,
- le 2-N-(2-butyloctyloxycarbonyl)-amino-1-éthanol,
- le 3-N-(2-éthylhexyloxycarbonyl)-amino-1,2-propanediol, et
- le 3-N-(2-butyloctyloxycarbonyl)-amino-1,2-propanediol.

Les composés de formule (I) peuvent être obtenus par réaction, dans un solvant, d'un aminoalcool de formule R₃-NH-A avec un composé de formule (II) : 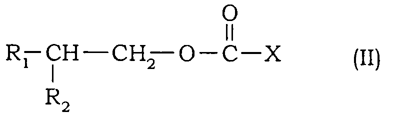 R₁, R₂, R₃, A ayant les même significations que celles données ci-dessus et X représentant un atome d'halogène, notamment un atome de chlore, ou un radical dérivé d'un azole, notamment un radical provenant d'un imidazole tel que celui de formule (III) :

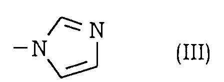

Comme solvant, on peut utiliser le dichlorométhane, le dichloro-1,2-éthane, le trichloro-1,1,1-éthane, le chloroforme, l'acétonitrile, le toluène, le dioxane, le tétrahydrofuranne, le diméthoxy-1,2-éthane, le cyclohexane, l'eau ou un mélange de ces solvants.
La réaction est effectuée à une température comprise entre -5°C et 50°C, de préférence inférieure à 10°C.
La réaction peut être effectuée en présence d'une base. Celle-ci peut être choisie parmi les hydroxydes de métaux alcalins ou alcalino-terreux, l'hydrogénocarbonate de sodium, les alcoolates de métaux alcalins, les hydrures alcalins, les amines tertiaires telles que la pyridine ou la triéthylamine.

Les composés de formule (I) peuvent être utilisés dans une composition cosmétique ou pharmaceutique, qui peut en plus comprendre un véhicule cosmétiquement acceptable tel que l'eau; les solvants organiques compatibles avec une application cosmétique tels que l'acétone, l'isopropanol, l'éthanol; les triglycérides d'acides gras à 6-24 atomes de carbone, les éthers de glycol, les esters de polyalkylèneglycols et les silicones volatiles.

Les composés de formule (I) peuvent être présents dans la composition à raison de 0,1 à 10% en poids de composé par rapport au poids total de la composition, et de préférence à raison de 0,5 à 5% en poids.

On a également constaté que les composés de formule (I) présentaient une grande compatibilité avec les tensioactifs habituellement utilisés dans le domaine cosmétique tels que le laurylsulfate de sodium ou le lauryléthersulfate de sodium. Il est ainsi possible de diminuer le caractère irritant pour la peau des tensioactifs usuels en les associant avec un des composés de formule (I).
On peut par exemple utiliser comme système tensioactif, un mélange comprenant du laurylsulfate de sodium et de la N-2-éthylhexyloxycarbonyl-N-méthyl-D-glucamine, de préférence dans un rapport 1/1, ou encore dans des proportions dans lesquelles le laurylsulfate de sodium est majoritaire.

La composition comprenant les composés de formule (I) peut se présenter sous forme de lotion aqueuse, de gel, d'émulsion, de crème, de mousse ou de pâte à appliquer sur la peau, les dents et/ou les cheveux.
En particulier, elle peut se présenter sous forme d'un shampooing, d'un gel douche, d'une pâte dentifrice, d'un lait ou d'une lotion démaquillante.

Selon sa destination, ladite composition peut également comprendre des corps gras, notamment les huiles naturelles ou synthétiques; des agents épaississants ou gélifiants tels que la cellulose ou ses dérivés, les polymères acryliques, les alginates, les gommes, les polyéthylèneglycols, les bentonites et les montmorillonites; des agents humectants tels que la glycérine et la triacétine; des agents antioxydants; des conservateurs.

L'invention est illustrée plus en détail dans les exemples suivants.

### Exemple 1

### Préparation de la N-2-éthylhexyloxycarbonyl-N-méthyl-D-glucamine

Dans un réacteur, on dissout 117 g (0,6 mole) de N-méthyl-D-glucamine dans un mélange de 800 ml d'eau et 400 ml de tétrahydrofuranne, puis on disperse 201,6 g (2,4 moles) d'hydrogénocarbonate de sodium.
En maintenant la température du mélange réactionnel à 5°C, on ajoute goutte-à-goutte 115,6 g (0,6 mole) de chloroformiate de 2-éthylhexyle puis on laisse réagir 3 heures sous agitation à 5°C et une nuit au repos à la température ambiante.
Le mélange réactionnel est alors filtré et concentré; le résidu pâteux obtenu est dissous dans 2 litres d'acétone puis filtré après refroidissement à 5°C. Le produit cristallisé recueilli est séché.
On obtient 105 g (rendement 50 %) de N-2-éthylhexyloxycarbonyl-N-méthyl-D-glucamine, dont le point de fusion est de 74°C.

### Exemple 2

### Préparation de la N-2-butyloctyloxycarbonyl-N-méthyl-D-glucamine

Le composé est préparé selon le même mode opératoire que l'exemple 1, en utilisant :
- 78 g (0,4 mole) de N-méthyl-D-glucamine
- 134,4 g (1,6 mole) de bicarbonate de soude
- 99,4 g (0,4 mole) de chloroformiate de 2-butyloctyle.
On obtient 62 g de N-2-butyloctyloxycarbonyl-N-méthyl-D-glucamine sous forme d'une poudre blanche dont le point de fusion est 77°C.

### Exemple 3

### Préparation de la N-2-éthylhexyloxycarbonyl-D-glucamine.

Le composé est préparé selon le même mode opératoire que l'exemple 1, en utilisant :
- 23,50 g (0,13 mole) de D-glucamine
- 43,70 g de bicarbonate de soude
- 25,04 g (0,13 mole) de chloroformiate de 2-éthylhexyle.
On obtient la N-2-éthylhexyloxycarbonyl-D-glucamine, dont le point de fusion est de 104°C, avec un rendement de 95%.

### Exemple 4

### Préparation de la N-2-butyloctyloxycarbonyl-D-glucamine.

Le composé est préparé selon le même mode opératoire que l'exemple 1, en utilisant :
- 47,00 g (0,26 mole) de D-glucamine
- 84 g d'hydrogénocarbonate de sodium, et
- 64,61 g (0,26 mole) de chloroformiate de butyloctyle.
On obtient la N-2-butyloctyloxycarbonyl-D-glucamine, dont le point de fusion est de 100,7°C, avec un rendement de 45%.

### Exemple 5

### Préparation du 2-N-(2-éthylhexyloxycarbonyl)-amino-1-éthanol.

Le composé est préparé selon le même mode opératoire que l'exemple 1, en utilisant :
- 21,35 g (0,35 mole) d'aminoéthanol,
- 67 g de bicarbonate de soude, et
- 38,50 g (0,2 mole) de chloroformiate de 2-éthylhexyle.
Après filtration du sel formé, on concentre sous vide et l'on obtient le composé sous forme d'huile incolore, avec un rendement de 98%.

### Exemple 6

### Préparation du 2-N-(2-butyloctyloxycarbonyl)-amino-1-éthanol.

Le composé est préparé selon le même mode opératoire que l'exemple 1, en utilisant :
- 18,3 g (0,3 mole) d'aminoéthanol,
- 67,2 g de bicarbonate de soude, et
- 49,7 g (0,2 mole) de chloroformiate de 2-butyloctyle.
Après filtration du sel formé, on concentre sous vide et l'on obtient le composé recherché sous forme d'huile incolore, avec un rendement de 99%.

### Exemple 7

### Préparation du 2-N-(2-éthylhexyloxycarbonyl)-amino-2-hydroxyméthyl-1,3-propanediol.

Dans un réacteur, on dissout 30 g de tris(hydroxyméthyl)aminométhane dans un mélange de 125 ml d'eau et 170 ml de tétrahydrofuranne, puis on disperse 84 g d'hydrogénocarbonate de sodium.
En maintenant la température du mélange réactionnel à 5°C, on ajoute goutte-à-goutte 62 g de chloroformiate de 2-éthylhexyle puis on laisse réagir 3 heures sous agitation à 5°C et une nuit au repos à la température ambiante.

Le mélange réactionnel est alors filtré et concentré; le résidu pâteux obtenu est dissous dans 800 ml d'acétonitrile puis porté sous agitation à 40°C pendant 1 h.
Le mélange est ensuite filtré puis concentré. Le résidu est purifié par chromatographie sur colonne de silice dans le dichlorométhane.
On obtient 14 g (rendement 20 %) de 2-N-(2-éthylhexyloxycarbonyl)-amino-2-hydroxyméthyl-1,3-propanediol sous forme d'une poudre blanche, dont le point de fusion est de 64°C.

### Exemple 8

### Préparation du 2-N-(2-butyloctyloxycarbonyl)-amino-2-hydroxyméthyl-1,3-propanediol.

Le composé est préparé selon le même mode opératoire que l'exemple 7, en utilisant :
- 30 g de tris(hydroxyméthyl)aminométhane
- 84 g d'hydrogénocarbonate de sodium, et
- 62 g de chloroformiate de 2-butyloctyle.

Le produit brut est recristallisé dans l'éthanol bouillant.
On obtient 22 g (rendement 55 %) de 2-N-(2-butyloctyloxycarbonyl)-amino-2-hydroxyméthyl-1,3-propanediol sous forme d'une poudre blanche, dont le point de fusion est de 67°C.

### Exemple 9

### Préparation du 3-N-(2-éthylhexyloxycarbonyl)-amino-1,2-propanediol.

Dans un réacteur, on dissout 22,8 g de 3-amino-1,2-propanediol dans un mélange de 120 ml d'eau et 180 ml de tétrahydrofuranne, puis on disperse 84 g d'hydrogénocarbonate de sodium.
En maintenant la température du mélange réactionnel à 5°C, on ajoute goutte-à-goutte 48 g de chloroformiate de 2-éthylhexyle puis on laisse réagir 3 heures sous agitation à 5°C et une nuit au repos à la température ambiante.
Le mélange réactionnel est alors filtré et concentré; le résidu pâteux obtenu est dissous dans 800 ml d'acétone puis porté sous agitation à 40°C pendant 1 h. Le mélange est ensuite filtré puis concentré.
On obtient 60 g (rendement 97 %) de 3-N-(2-éthylhexyloxycarbonyl)-amino-1,2-propanediol sous forme d'une huile translucide.

### Exemple 10

### Préparation du 3-N-(2-butyloctyloxycarbonyl)-amino-1,2-propanediol.

Le composé est préparé selon le même mode opératoire que l'exemple 9, en utilisant :
- 22,8 g de 3-amino-1,2-propanediol
- 84 g d'hydrogénocarbonate de sodium, et
- 62 g de chloroformiate de 2-butyloctyle.
On obtient 74 g (rendement 97 %) de 3-N-(2-butyloctyloxycarbonyl)amino-1,2-propanediol sous forme d'une huile translucide.

### Exemple 11

On compare la tension superficielle au plateau (γ plateau) et la concentration micellaire critique (CMC) pour
. la N-2-éthylhexyloxycarbonyl-N-méthyl-D-glucamine, composé selon l'invention, préparé à l'exemple 1, et
. la N-octanoyl-N-méthyl-D-glucamine, vendu sous le nom de MEGA 8 par DOJlNDO, tensioactif commercialisé notamment dans le domaine cosmétique.

La tension superficielle au plateau et la CMC sont déduites par mesure de la tension de surface statique à l'aide d'un tensiomètre à anneau de type DU NOUY.

On obtient les résultats suivants:

| | γ plateau dyn/cm ou mN/m | CMC (mole/l) |
|---|---|---|
| Composé selon l'invention | 26,5 | 0,011 |
| MEGA 8 | 39 | 0,052 |

On constate donc que la tension superficielle du composé de formule (I) est nettement inférieure à celle du composé de l'état de la technique.
Le composé selon l'invention possède donc des propriétés de surface supérieures à celles du composé de l'état de la technique.

On constate également que la CMC du composé de formule (I) est environ 5 fois inférieure à celle du composé de l'état de la technique, d'où l'intérêt des composés selon l'invention qui possèdent de bonnes propriétés de surface même à concentration faible.

### Exemple 12

On compare le comportement cutané de deux composés en tant que tensioactifs:
. le laurylsulfate de sodium (ou LSNa), tensioactif usuel, et
. la N-2-éthylhexyloxycarbonyl-N-méthyl-D-glucamine, composé selon l'invention.

La comparaison est effectuée à l'aide du test décrit dans « An in vitro method for evaluation of the irritancy of anionic surfactants » de Anavkar et al, publiée dans JAOCS, volume 66, n°9 (1989), pp. 1386-1389.

Le principe de ce test consiste à déterminer la capacité, pour les composés considérés, d'extraire un colorant présent à l'intérieur d'une masse protéique.
On prépare une matrice protéique (gel de gélatine) contenant un colorant particulier (le CBB ou Comassie Brilliant Blue). On verse sur le gel obtenu une solution de composé considéré et l'on agite à 105 tours/minute, 20 °C pendant 30 minutes.
On mesure ensuite la quantité de colorant extraite, par spectrophotométrie à 590 nm.
Cette quantité est proportionnelle au caractère irritant du tensioactif pour le support sur lequel il est appliqué (peau, cuir chevelu, etc.).
Ainsi, plus l'absorbance mesurée est faible, meilleur est le comportement cutané du composé testé.

On obtient les résultats suivants :

| Composé considéré | Absorbance à 590 nm |
|---|---|
| Solution aqueuse contenant 1% en poids de LSNa | 2,459 |
| Solution aqueuse contenant 1% en poids du composé selon l'invention | 0,552 |
| Solution aqueuse contenant 1% en poids de LSNa et 1% en poids du composé selon l'invention | 0,261 |

On peut donc considérer que le composé selon l'invention est beaucoup moins irritant vis-à-vis de la peau qu'un tensioactif usuel, le LSNa.
De plus, l'association du composé selon l'invention avec le laurylsulfate de sodium permet de diminuer l'agressivité de ce dernier.

### Exemple 13

On prépare une pâte dentifrice ayant la composition suivante:

| | |
|---|---|
| . silice amorphe précipitée | 20 g |
| . dioxyde de titane | 0,5 g |
| . carboxyméthylcellulose | 1,4 g |
| . sorbitol en solution aqueuse à 70% de matière active | 32 g |
| . laurylsulfate de sodium (93% de matière active) | 1 g |
| . N-2-éthylhexyloxycarbonyl-N-méthyl-D-glucamine | 1 g |
| . fluorure de sodium | 0,11 g |
| . monofluorophosphate de sodium | 0,38 g |
| . édulcorant, conservateur et arôme | qs |
| . eau | qsp 100 g |

### Exemple 14

On prépare une pâte dentifrice ayant la composition suivante:

| | |
|---|---|
| . silice amorphe précipitée | 20 g |
| . dioxyde de titane | 0,5 g |
| . carboxyméthylcellulose | 1,4 g |
| . sorbitol en solution aqueuse à 70% de matière active | 32 g |
| . N-2-éthylhexyloxycarbonyl-N-méthyl-D-glucamine | 1 g |
| . fluorure de sodium | 0,11 g |
| . monofluorophosphate de sodium | 0,38 g |
| . édulcorant, conservateur et arôme | qs |
| . eau | qsp 100 g |

### Exemple 15

On prépare un gel douche ayant la composition suivante:

| | |
|---|---|
| . laurylsulfate de triéthanolamine (40% de matière active) | 30 g |
| . cocoyl bétaïne (32% de matière active) | 10 g |
| . laurylsulfate de sodium (93% de matière active) | 5,4 g |
| . N-2-éthylhexyloxycarbonyl-N-méthyl-D-glucamine | 5 g |
| . conservateur, parfum, colorant | qs |
| . NaOH ou HCl | qs pH 7,2 |
| . eau | qsp 100 g |

On obtient un gel douche possédant de bonnes propriétés cosmétiques.

### Exemple 16

On prépare un gel douche ayant la composition suivante:

| | |
|---|---|
| . laurylsulfate de triéthanolamine (40% de matière active) | 30 g |
| . cocoyl bétaïne (32% de matière active) | 10 g |
| . N-2-éthylhexyloxycarbonyl-N-méthyl-D-glucamine | 5 g |
| . conservateur, parfum, colorant | qs |
| . NaOH ou HCl | qs pH 7,2 |
| . eau | qsp 100 g |

On obtient un gel douche possédant de bonnes propriétés cosmétiques.

### Exemple 17

On prépare un lait démaquillant ayant la composition suivante:

| | |
|---|---|
| . palmitate d'isopropyle | 5 g |
| . copolymère carboxyvinylique (Pemulen TR2 de Goodrich) | 0,1 g |
| . N-2-éthylhexyloxycarbonyl-N-méthyl-D-glucamine | 3 g |
| . NaOH | 0,02 g |
| . conservateur, parfum | qs |
| . eau | qsp 100 g |

On obtient un lait démaquillant ayant de bonnes propriétés cosmétiques.

### Exemple 18

On prépare une lotion détergente douce ayant la composition suivante:

| | |
|---|---|
| . N-2-éthylhexyloxycarbonyl-N-méthyl-D-glucamine | 3 g |
| . laurate de sorbitan (20 OE) | 2 g |
| . gomme de xanthane | 0,15 g |
| . butylène 1,3-glycol | 4 g |
| . conservateur, parfum | qs |
| . eau | qsp 100 g |

On obtient une lotion détergente peu irritante pour la peau.

## Revendications

1. Utilisation en tant que tensioactif dans une composition cosmétique ou pharmaceutique d'un composé de formule (I) : 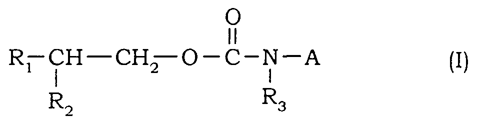 dans laquelle:
- R₁ représente un radical alkyle, saturé ou insaturé, linéaire ou ramifié, ayant de 4 à 6 atomes de carbone,
- R₂ représente un radical alkyle, saturé ou insaturé, linéaire ou ramifié, ayant de 2 à 4 atomes de carbone,
- R₃ représente un atome d'hydrogène ou un radical alkyle, saturé ou insaturé, linéaire ou ramifié, ayant de 1 à 6 atomes de carbone, et
- A représente un groupement hydrophile non ionique.

2. Utilisation selon la revendication 1, dans laquelle A représente un radical :
―(CH₂)ₙ―(CHOH)ₘ―Z
dans lequel n est un nombre entier égal à 0 ou 1, m est un nombre entier compris entre 0 et 4, et Z est un radical alkyle hydroxylé ayant de 1 à 4 atomes de carbone.

3. Utilisation selon la revendication 2, dans laquelle Z est choisi dans le groupe constitué par :
―CH₂OH et ―C(CH₂OH)₃

4. Utilisation selon l'une quelconque des revendications précédentes, dans laquelle le composé de formule (I) est choisi parmi :
- la N-2-éthylhexyloxycarbonyl-N-méthyl-D-glucamine,
- la N-2-butyloctyloxycarbonyl-N-méthyl-D-glucamine,
- la N-2-éthylhexyloxycarbonyl-D-glucamine,
- la N-2-butyloctyloxycarbonyl-D-glucamine,
- le 2-N-(2-éthylhexyloxycarbonyl)-amino-2-hydroxyméthyl-1,3-propanediol,
- le 2-N-(2-butyloctyloxycarbonyl)-amino-2-hydroxyméthyl-1,3-propanediol,
- le 2-N-(2-éthylhexyloxycarbonyl)-amino-1-éthanol,
- le 2-N-(2-butyloctyloxycarbonyl)-amino-1-éthanol,
- le 3-N-(2-éthylhexyloxycarbonyl)-amino-1,2-propanediol, et
- le 3-N-(2-butyloctyloxycarbonyl)-amino-1,2-propanediol.

5. Utilisation selon l'une quelconque des revendications précédentes, dans laquelle le composé de formule (I) est présent à une concentration comprise entre 0,1-10 % en poids par rapport au poids total de la composition.

6. Utilisation selon l'une quelconque des revendications précédentes, dans laquelle le composé de formule (I) est présent à une concentration comprise entre 0,5-5 % en poids par rapport au poids total de la composition.

7. Utilisation selon l'une quelconque des revendications précédentes, dans laquelle le composé de formule (I) est utilisé en association avec un second tensioactif.

8. Utilisation selon la revendication 7, dans laquelle le second tensioactif est choisi parmi le laurylsulfate de sodium et le lauryléthersulfate de sodium.

9. Utilisation selon l'une quelconque des revendications précédentes, dans une composition se présentant sous forme de lotion aqueuse, de gel, d'émulsion, de crème, de mousse ou de pâte à appliquer sur la peau, les dents et/ou les cheveux.

10. Utilisation selon l'une quelconque des revendications précédentes dans une composition se présentant sous forme d'un shampooing, d'un gel douche, d'une pâte dentifrice, d'un lait ou d'une lotion démaquillante.

## Claims

1. Use, as surfactant, in a cosmetic or pharmaceutical composition, of a compound of formula (I): 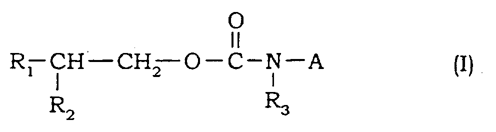 in which:
- R₁ represents a linear or branched, saturated or unsaturated alkyl radical having 4 to 6 carbon atoms,
- R₂ represents a linear or branched, saturated or unsaturated alkyl radical having 2 to 4 carbon atoms,
- R₃ represents a hydrogen atom or a linear or branched, saturated or unsaturated alkyl radical having 1 to 6 carbon atoms, and
- A represents a nonionic hydrophilic group.

2. Use according to Claim 1, in which A represents a radical:
-(CH₂)ₙ-(CHOH)ₘ-Z
in which n is an integer equal to 0 or 1, m is an integer between 0 and 4, and Z is a hydroxylated alkyl radical having from 1 to 4 carbon atoms.

3. Use according to Claim 2, in which Z is chosen from the group consisting of:
-CH₂OH and -C(CH₂OH)₃

4. Use according to any one of the preceding claims, in which the compound of formula (I) is chosen from:
- N-2-ethylhexyloxycarbonyl-N-methyl-D-glucamine,
- N-2-butyloctyloxycarbonyl-N-methyl-D-glucamine,
- N-2-ethylhexyloxycarbonyl-D-glucamine,
- N-2-butyloctyloxycarbonyl-D-glucamine,
- 2-N-(2-ethylhexyloxycarbonyl)amino-2-hydroxymethyl-1,3-propanediol,
- 2-N-(2-butyloctyloxycarbonyl)amino-2-hydroxymethyl-1,3-propanediol,
- 2-N-(2-ethylhexyloxycarbonyl)amino-1-ethanol,
- 2-N-(2-butyloctyloxycarbonyl)amino-1-ethanol,
- 3-N-(2-ethylhexyloxycarbonyl)amino-1,2-propanediol, and
- 3-N-(2-butyloctyloxycarbonyl)amino-1,2-propanediol.

5. Use according to any one of the preceding claims, in which the compound of formula (I) is present at a concentration of between 0.1-10 % by weight relative to the total weight of the composition.

6. Use according to any one of the preceding claims, in which the compound of formula (I) is present at a concentration of between 0.5-5 % by weight relative to the total weight of the composition.

7. Use according to any one of the preceding claims, in which the compound of formula (I) is used in combination with a second surfactant.

8. Use according to Claim 7, in which the second surfactant is chosen from sodiumlauryl sulphate and sodiumlauryl ether sulphate.

9. Use according to any one of the preceding claims, in a composition provided in the form of an aqueous lotion, a gel, an emulsion, a cream, a foam or a paste to be applied to the skin, the teeth and/or the hair.

10. Use according to any one of the preceding claims, in a composition provided in the form of a shampoo, a shower gel, a toothpaste, a milk or a make-up removing lotion.

## Patentansprüche

1. Verwendung als grenzflächenaktiver Stoff in einer kosmetischen oder pharmazeutischen Zusammensetzung einer Verbindung der Formel (I), 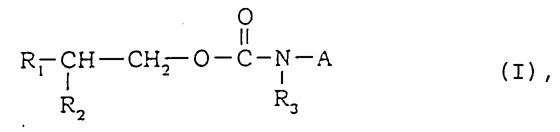 worin bedeuten:
- R₁ einen geradkettigen oder verzweigten, gesättigten oder ungesättigten Alkylrest mit 4 bis 6 Kohlenstoffatomen,
- R₂ einen geradkettigen oder verzweigten, gesättigten oder ungesättigten Alkylrest mit 2 bis 4 Kohlenstoffatomen,
- R₃ Wasserstoff oder einen geradkettigen oder verzweigten, gesättigten oder ungesättigten Alkylrest mit 1 bis 6 Kohlenstoffatomen und
- A eine nichtionische hydrophile Gruppe.

2. Verwendung nach Anspruch 1, wobei A eine Gruppe
-(CH₂)ₙ -(CHOH)ₘ -Z
darstellt, in der n gleich 0 oder 1 ist, m eine ganze Zahl von 0 bis 4 darstellt und Z ein hydroxylierter Alkylrest mit 1 bis 4 Kohlenstoffatomen ist.

3. Verwendung nach Anspruch 2, wobei Z ausgewählt wird unter -CH₂OH und -C(CH₂OH)₃.

4. Verwendung nach einem der vorhergehenden Ansprüchen, wobei die Verbindung der Formel I ausgewählt wird unter
- *N*-2-Ethylhexyloxycarbonyl-*N*-methyl-D-glucamin,
- *N*-2-Butyloctyloxycarbonyl-*N*-methyl-D-glucamin,
- *N*-2-Ethylhexyloxycarbonyl-D-glucamin,
- *N*-2-Butyloctyloxycarbonyl-D-glucamin,
- 2-*N*-(2-Ethylhexyloxycarbonyl)-amino-2-hydroxymethyl-1,3-propandiol,
- 2-*N*-(2-Butyloctyloxycarbonyl)-amino-2-hydroxymethyl-1,3-propandiol,
- 2-*N*-(2-Ethylhexyloxycarbonyl)-amino-1-ethanol,
- 2-*N*-(2-Butyloctyloxycarbonyl)-amino-1-ethanol,
- 3-*N*-(2-Ethylhexyloxycarbonyl)-amino-1,2-propandiol und
- 3-*N*-(2-Butyloctyloxycarbonyl)-amino-1,2-propandiol.

5. Verwendung nach einem der vorhergehenden Ansprüche, wobei die Verbindung der Formel (I) in einer Konzentration von 0,1 bis 10 Gew.-%, bezogen auf das Gesamtgewicht der Zusammensetzung, enthalten ist.

6. Verwendung nach einem der vorhergehenden Ansprüche, wobei die Verbindung der Formel (I) in einer Konzentration von 0,5 bis 5 Gew.-%, bezogen auf das Gesamtgewicht der Zusammensetzung, enthalten ist.

7. Verwendung nach einem der vorhergehenden Ansprüche, wobei die Verbindung der Formel (I) in Kombination mit einem zweiten grenzflächenaktiven Stoff verwendet wird.

8. Verwendung nach Anspruch 7, wobei der zweite grenzflächenaktive Stoff unter Natriumlaurylsulfat und Natriumlaurylethersulfat ausgewählt wird.

9. Verwendung nach einem der vorhergehenden Ansprüche, wobei die Zusammensetzung in Form einer wäßrigen Lotion, eines Gels, einer Emulsion, einer Creme, eines Schaums oder einer Paste vorliegt, wobei jede dieser Formen dafür bestimmt ist, auf die Haut, die Zähne und/oder die Haare aufgebracht zu werden.

10. Verwendung nach einem der vorhergehenden Ansprüche, wobei die Zusammensetzung in Form eines Haarwaschmittels, eines Duschgels, einer Zahncreme oder einer Milch oder Lotion zum Anschminken vorliegt.
